Europäisches Patentamt

European Patent Office

Office européen des brevets

(11) Veröffentlichungsnummer: **0 141 089**
**A2**

## (12) EUROPÄISCHE PATENTANMELDUNG

(21) Anmeldenummer: **84109652.2**

(22) Anmeldetag: **14.08.84**

(51) Int. Cl.⁴: **G 01 N 27/12**
**G 01 N 33/00**

(30) Priorität: **30.08.83 CH 4736/83**

(43) Veröffentlichungstag der Anmeldung:
**15.05.85 Patentblatt 85/20**

(84) Benannte Vertragsstaaten:
**BE CH DE FR GB IT LI**

(71) Anmelder: **CERBERUS AG**
**Alte Landstrasse 411**
**CH-8708 Männedorf(CH)**

(72) Erfinder: **Forster, Martin, Dr. sc. nat.**
**Sonnenbergstrasse 16**
**CH-8645 Jona(CH)**

(74) Vertreter: **Tiemann, Ulrich, Dr.-Ing.**
**c/o Cerberus AG Patentabteilung Alte Landstrasse 411**
**CH-8708 Männedorf(CH)**

(54) **Vorrichtung zur selektiven Bestimmung der Bestandteile von Gasgemischen.**

(57) Ein Gassensor (6), welcher mehrere Sensorelemente (5) enthält, die bei Einwirkung von Gasen ihre elektrische Leitfähigkeit ändern, kann zur selektiven Bestimmung der Bestandteile von Gasgemischen verwendet werden, wenn die einzelnen Sensorelemente (5) auf einem hitzebeständigen, wärmeisolierenden Substrat (10) angeordnet werden und die einzelnen Sensorelemente (5) durch die Wahl unterschiedlicher Materialien (1) und/oder der Betriebstemperatur zur Bestimmung unterschiedlicher Bestandteile des Gasgemischs sensibilisiert werden. Gemäss bevorzugten Ausführungsformen werden die Bauteile der Sensorelemente (5) so angeordnet, dass das zu untersuchende Gasgemisch in einem gerichteten Strom an den Sensorelementen (5) vorbeigeleitet wird. Durch eine abgestuft erhöhte Temperatur der Sensorelemente (5) in Richtung des Gasstroms kann einer weitere Steigerung der Selektivität des Gassensors (6) erreicht werden.

Fig 1

Vorrichtung zur selektiven Bestimmung der Bestandteile von

Gasgemischen

---

Die Erfindung geht aus von einer Vorrichtung zur selektiven Bestimmung der Bestandteile von Gasgemischen der im Oberbegriff des Patentanspruchs 1 definierten Gattung. Eine solche Vorrichtung ist an der 32. Konferenz für elektronische Bauelemente, die vom 10. bis 12 Mai 1980 in San Diego, Kalifornien, USA stattfand, vorgestellt worden.

Gassensoren werden seit einiger Zeit für verschiedene Zwecke wie z.B. Umweltschutz, Garagen-Ueberwachung, Brandschutz und Explosionsschutz verwendet. Hierzu benützt man billige Sensoren aus Metalloxiden, deren elektrische Leitfähigkeit von der Konzentration der zu detektierenden Gase in der umgebenden Luft abhängt. Aus der Aenderung der elektrischen Leitfähigkeit kann auf die Konzentration an zu detektierendem Gas geschlossen werden. Zur Durchführung des Gasnachweises mit diesen Sensoren muss der Sensor auf eine Temperatur von ca. 450°C gebracht werden. Die bekannten Gassensoren bestehen aus feingemahlenem Metalloxidpulver, das auf einen mit Elektroden versehenen Träger aufgesintert ist und haben nur eine sehr geringe Selektivität in ihrer Gasdetektion. Die mit diesen Gassensoren erhaltenen Werte lassen keinen Schluss darauf zu, um welche Gaskomponente es sich innerhalb eines Gasgemisches handelt.

Da die einzelnen Bestandteile von Gasgemischen eine stark unterschiedliche Leitfähigkeitsänderung der Gassensoren hervorrufen, d.h. die Gassensoren eine unterschiedliche Empfindlichkeit gegenüber den Bestandteilen des Gasgemisches aufweisen, ist es wünschenswert, Angaben über die Art des Gases zu erhalten. Bei der Versorgung von Wohngebieten mit Stadtgas oder Erdgas ist es wichtig, den Austritt von Gas aus Leckstellen und toxisches Kohlenmonoxid, das durch unvollständige Verbrennungsvorgänge entsteht, schnell und sicher nachzuweisen. Ein häufiges Störgas ist hierbei Aethylalkoholdampf, der in Küchen etc. häufig auftritt, da die Gassensoren, die auf Kohlenmonoxid und Kohlenwasserstoffe empfindlich sind, auf Aethylalkoholdampf ebenfalls ansprechen.

An der 32. Konferenz über elektronische Bauelemente wurde daher ein Gassensor vorgeschlagen, bei dem drei aus unterschiedlichen Materialien bestehende Gassensoren vorgesehen sind, von denen zwei zum Nachweis von Methan (Hauptbestandteil im Erdgas), Wasserstoff und/oder Kohlenmonoxid (Bestandteile im Stadtgas und durch unvollständige Verbrennung entstanden) dienen. Da beide Sensorelemente auch auf Alkoholdampf (Störgas) ansprechen, wird die Konzentration an Alkoholdampf mit einem dritten auf Aethylalkoholdampf spezifisch reagierenden Gassensor gemessen, und dieser Wert wird von den mit den beiden anderen Sensorelementen bestimmten Konzentrationen an Methan und Kohlenmonoxid abgezogen. Dieser Gasdetektor weist den Nachteil auf, dass er lediglich auf zwei Bestandteile von Gasgemischen reagiert, wobei er einen weiteren spezifischen Bestandteil als Störfaktor ausschliessen kann.

Die vorliegende Erfindung hat sich die Aufgabe gestellt, eine Vorrichtung der eingangs erwähnten Art zu schaffen, welche die Nachteile der bekannten Vorrichtungen vermeidet und eine selektive Bestimmung verschiedener Bestandteile von Gasgemischen gestattet.

Diese Aufgabe wird erfindungsgemäss durch die im kennzeichnenden Teil des Patentanspruchs 1 definierten Merkmale gelöst. Bevorzugte Ausführungsformen und Weiterbildungen der erfindungsgemässen Vorrichtung sind in den abhängigen Ansprüchen definiert.

Gemäss einer bevorzugten Ausführungsform der erfindungsgemässen Vorrichtung sind die Sensorelemente und die gegebenenfalls vorhandenen Heizvorrichtungen so angeordnet, dass das zu untersuchende Gasgemisch mit einem gerichteten Strom an den Sensorelementen vorbeigeleitet wird. Gemäss einer besonders bevorzugten Ausführungsform kann dafür eine gasdichte Schicht vorgesehen sein, die das zu untersuchende Gasgemisch so lenkt, dass es vorzugsweise die Sensorelemente nacheinander überstreicht.

Gemäss einer weiteren Ausgestaltung weisen die Sensorelemente eine unterschiedliche Betriebstemperatur auf, wobei vorzugsweise jeweils das in Richtung des Gasstroms gesehen vornliegende Sensorelement eine niedrigere Temperatur aufweist als das dahinterliegende Sensorelement.

Die Sensorelemente können auch in einem zusammenhängenden Block angeordnet sein, wobei in Richtung des Gasstroms ein Temperaturgradient vorhanden ist, d.h. die Temperatur in Richtung des Gasstroms allmählich ansteigt. Auf dem Sensorblock sind dabei in Reihe hintereinander Elektroden angeordnet, die jeweils einem unterschiedlichen Temperaturbereich entsprechen. An unterschiedlich heissen Teilen des Sensorblocks werden unterschiedliche Gase detektiert, wobei die Leitfähigkeitsänderung durch die entsprechenden zugeordneten Elektroden der Auswerteschaltung zugeführt werden.

Im folgenden werden anhand der Figuren bevorzugte Ausführungsbeispiele der Erfindung näher erläutert. Es zeigen:

Figur 1 einen Schnitt durch einen Gassensor.

Figur 2 eine Draufsicht auf einen Gassensor wie er in Figur 1 im Querschnitt dargestellt ist,

Figuren 3 bis 5 verschiedene Ausführungsformen von Gassensoren mit mehreren Sensorelementen und

Figuren 6 bis 9 Ausführungsformen eines Gassensors, bei denen das Gasgemisch in einem gerichteten Strom an den Sensorelementen vorbeigeleitet wird.

Die Sensorelemente 5 der Gassensoren 6 sind aus einem Material 1 hergestellt das in den Schweizerischen Patenten Nr.         (Patentgesuch 4737/83-9) und         (Patentgesuch Nr. 4738/83-0) beschrieben ist und das auch im Handel erhältlich ist.

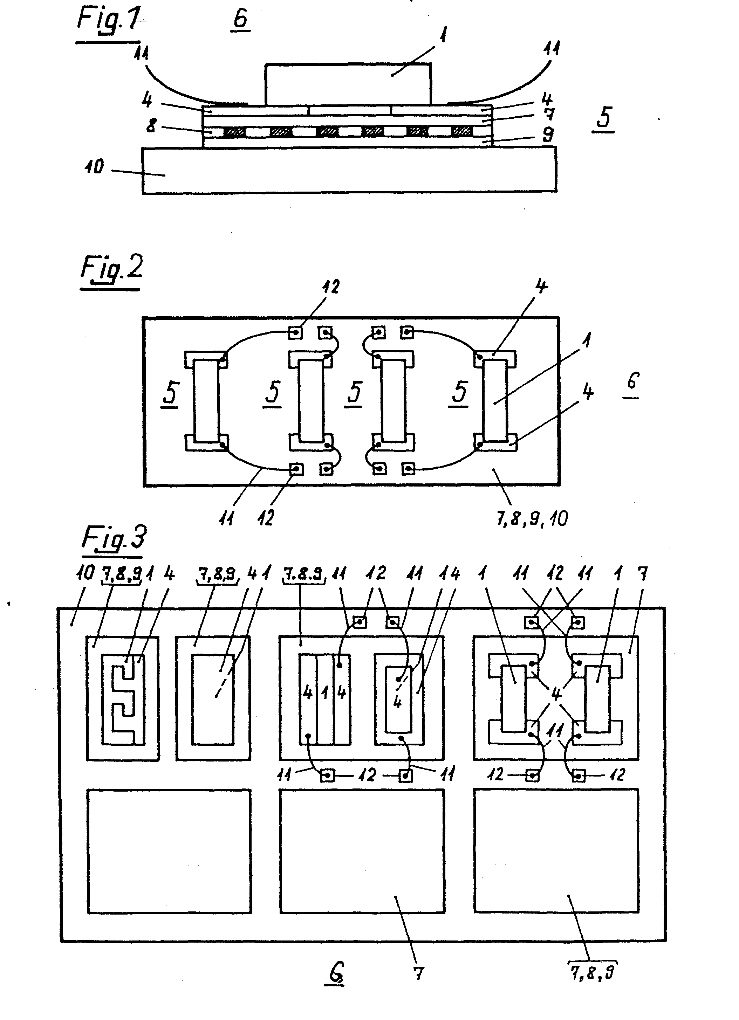

Die Figur 1 zeigt einen Querschnitt durch einen Gassensor 6 an einer Stelle, an welcher ein Sensorelement 5 angeordnet ist. Der Gassensor 6 weist mehrere solche Sensorelemente 5 auf. Das Material 1, das bei Einwirkung von Gasen seine elektrische Leitfähigkeit ändert, ist an seinen Enden mit zwei Elektroden 4 verbunden, die über elektrische Leitungen 11 mit einer nicht dargestellten Auswerteschaltung verbunden sind. Unterhalb des Materials 1 befindet sich die Heizvorrichtung 8, die zwischen zwei isolierenden Schichten 7 und 9 angeordnet ist. Die Heizvorrichtung 8 kann aus üblichen Heizdrähten bestehen oder eine Schicht aus Platin, Iridium oder ihren Legierungen sein mit einem grossen Temperaturkoeffizienten der elektrischen Leitfähigkeit, so dass gleichzeitig die Bestimmung der Heiztemperatur durch Messung des Widerstands der Heizvorrichtung erfolgen kann. Durch diese Heizvorrichtung wird das Material 1 des Sensorelementes 5 auf eine vorbestimmte Betriebstemperatur aufgeheizt. Die einzelnen Sensorelemente 5 sind auf einem hitzebeständigen, wärmeisolierenden Substrat 10 angeordnet und bilden zusammen den Gassensor 6. Der Gassensor 6 kann sich in einem nicht gezeichneten Gehäuse befinden, das an dem zu überwachenden Ort angebracht wird.

Die Heizvorrichtung 8 wird über nicht dargestellte Zuleitungsdrähte mit einer Stromquelle verbunden. In der Auswerteschaltung sind Schaltelemente vorhanden, die dafür sorgen, dass die vorbestimmte Betriebstemperatur, die im Bereich von Zimmertemperatur bis 450°C liegen kann, eingestellt und konstant gehalten wird. Dabei können die verschiedenen Sensorelemente 5 die aus den gleichen oder unterschiedlichen Materialien bestehen auf die gleiche Betriebstemperatur eingestellt werden, oder es kann jedes der Sensorelemente 5 zum Nachweis unterschiedlicher Bestandteile von Gasgemischen auf eine andere Temperatur in dem genannten Bereich erhitzt werden.

Um den Stromverbrauch des Gassensors 6 möglichst gering zu halten, werden die Abmessungen der Bauelemente möglichst klein gehalten. Da die Sensorelemente 5 nicht wie bei den Gassensoren des Standes der Technik an den Zuleitungsdrähten aufgehängt sind, sondern sich auf dem stabilen hitzebeständigen, wärmeisolierenden Substrat 10 befinden, können die Zuleitungsdrähte einen sehr geringen Querschnitt aufweisen. Ein Durchmesser von 30 µm ist beispielsweise völlig ausreichend, wodurch die Wärmeableitung weiter reduziert wird. Die räumlichen Abmessungen des Gassensors 6 der Figur 1 beträgt 5 mm Höhe, 3 mm Breite und 3 mm Länge. Wegen dieser ausserordentlich geringen Abmessungen können die Gassensoren auch an schwer zugänglichen Orten angebracht werden.

Ist anzunehmen, dass nur ein einziges gefährliches Gas auftritt, so müssen nicht mehrere Sensorelemente 5 vorhanden sein. Für diesen Fall genügt ein einziges Sensorelement 5, das mit seiner zwischen zwei elektrisch isolierenden Schichten 7 und 9 befindlichen Heizvorrichtung 8 auf einem hitzebeständigen, wärmeisolierenden Substrat 10 angeordnet ist. Die Heizvorrichtung 8 bringt das Material 1 des Gassensors 6 auf eine Temperatur, die optimal abgestimmt ist zum Nachweis des einen gefährlichen Gases. Durch die Verwendung des wärmeisolierenden Substrats 10 wird der Betrieb eines solchen erfindungsgemässen Gassensors 6 sehr ökonomisch, da nur eine geringe Heizleistung notwendig ist, die bei einer Temperatur des Materials 1 von 360°C nur ca. 300mW beträgt.

In Figur 2 ist ein Gassensor 6, der in Figur 1 im Querschnitt dargestellt ist, in der Draufsicht gezeichnet. Die elektrischen Leitungen 11 der Elektroden 4 sind zu Kontaktpunkten 12 geführt, die auf der Isolationsschicht 7 befestigt sind. Die elektrischen Kontaktpunkte 12 sind über nicht gezeichnete Leitungen mit der Auswerteschaltung verbunden.

Die Sensorelemente 5 können durch Schaltelemente der Auswerteschaltung alle auf die gleiche Betriebstemperatur aufgeheizt werden oder sie können jeweils eine unterschiedliche Betriebstemperatur aufweisen. Es ist auch möglich, sämtliche Sensorelemente 5 bei Zimmertemperatur zu betreiben. Dann können die Heizschicht 8 und die elektrisch isolierenden Schichten 7 und 9 wegfallen.

Bei der Einwirkung eines Gasgemischs auf die Sensorelemente 5 eines Gassensors 6 ändern sich die elektrischen Leitfähigkeiten der einzelnen Sensorelemente 5 in Abhängigkeit von der Konzentration der Bestandteile. Durch Auswahl des Materials 1 und/oder der Betriebstemperatur der Sensorelemente 5 wird die Empfindlichkeit der einzelnen Sensorelemente 5 auf einen anderen Bestandteil des Gasgemischs eingestellt. Je nach der Zusammensetzung der zu untersuchenden Gasgemische werden die Materialien 1 und die Betriebstemperaturen der Sensorelemente 5 so eingestellt, dass eine gute Selektivität zwischen den einzelnen Bestandteilen gewährleistet ist. Die Aenderung der elektrischen Leitfähigkeit, die an den Elektroden 4 auftritt, wird über die elektrischen Leitungen 11 in der Auswerteschaltung gemessen.

Die Zahl der Sensorelemente 5 hängt von der Anzahl der Bestandteile ab, die in Gasgemischen bestimmt werden sollen. Die in Figur 2 dargestellte Vorrichtung ist beispielsweise zum Nachweis vier verschiedener Bestandteile geeignet.

Wenn eines der Sensorelemente 5 für mehrere Bestandteile des zu untersuchenden Gasgemischs empfindlich sein soll, d.h. wenn ein Störgas auftritt, kann die Empfindlichkeit eines Sensorelementes 5 so eingestellt werden, dass es auf das Störgas anspricht und die Auswerteschaltung kann in diesem Fall so ausgelegt werden, dass die Konzentration des Störgases rechnerisch eliminiert wird, so dass der gewünschte Bestandteil des Gasgemischs bestimmt werden kann.

Figur 3 zeigt in Draufsicht eine weitere Ausgestaltung eines Gassensors 6, wobei zur Erläuterung der Vielfältigkeit der Möglichkeiten in den einzelnen Bereichen unterschiedliche Sensorelemente 5 dargestellt sind. Wegen der besseren Uebersichtlichkeit sind nur in der oberen Reihe der Figur 3 die Sensorelemente 5 eingezeichnet.

In der oberen Reihe rechts ist eine Ausgestaltung dargestellt, bei der eine Heizvorrichtung nicht vorgesehen ist, da diese Sensorelemente 5 bei Zimmertemperatur betrieben werden. Es entfällt daher die zweite elektrisch isolierende Schicht und die erste elektrisch isolierende Schicht 7 kann direkt auf dem hitzebständigen, wärmeisolierenden Substrat 10 aufgebracht werden. Die elektrischen Leitungen 11 verbinden die Elektroden 4 mit den elektrischen Kontaktpunkten 12, die ebenfalls auf dem Substrat 10 befestigt sind. Von den Kontaktpunkten 12 gehen nicht dargestellte elektrische Leitungen zu einer Auswerteschaltung.

In der oberen Reihe der Figur 3 sind weitere Ausgestaltungen der Sensorelemente 5, beispielsweise mit verschieden geformten Elektroden 4, dargestellt. Bei der in der Mitte der oberen Reihe dargestellten Ausführungsform findet sich unter der Isolationsschicht 7 eine mehreren Sensorelementen 5 gemeinsame Heizschicht 8 und die elektrisch isolierende Schicht 9, die auf dem hitzebeständigen, wärmeisolierenden Substrat 10 befestigt ist. In diesem Fall werden beide Sensorelemente 5 auf die gleiche Betriebstemperatur aufgeheizt und konstant gehalten. Die Anpassung der Empfindlichkeit zur Bestimmung unterschiedlicher Bestandteile von Gasgemischen erfolgt durch eine unterschiedliche Ausbildung des Materials 1 der Sensorelemente 5.

In der oberen Reihe links der Figur 3 ist eine weitere Ausgestaltung der Sensorelemente 5 dargestellt, bei welcher jedes Sensorelement 5 eine eigene Heizschicht 8 aufweist und daher sind auch getrennte elektrisch isolierende Schichten 7 bzw. 9 vorgesehen. Die Ausgestaltung der übrigen Bauelemente ist wie oben angegeben, d.h. die Sensorelemente 5 befinden sich auf einem hitzebeständigen, wärmeisolierenden Substrat 10,
die Elektroden 4 sind über elektrische Leitungen 11 mit
elektrischen Kontaktpunkten 12 verbunden, die ihrerseits über
nicht dargestellte Leitungen mit einer Auswerteschaltung verbunden sind. Mit dem in Figur 3 dargestellten Gassensor 6
können zwölf verschiedene Gase detektiert werden. Die Anzahl
der Sensorelemente 5 kann der Anzahl der zu bestimmenden Bestandteile von Gasgemischen angepasst werden, d.h. höher oder
niedriger sein.

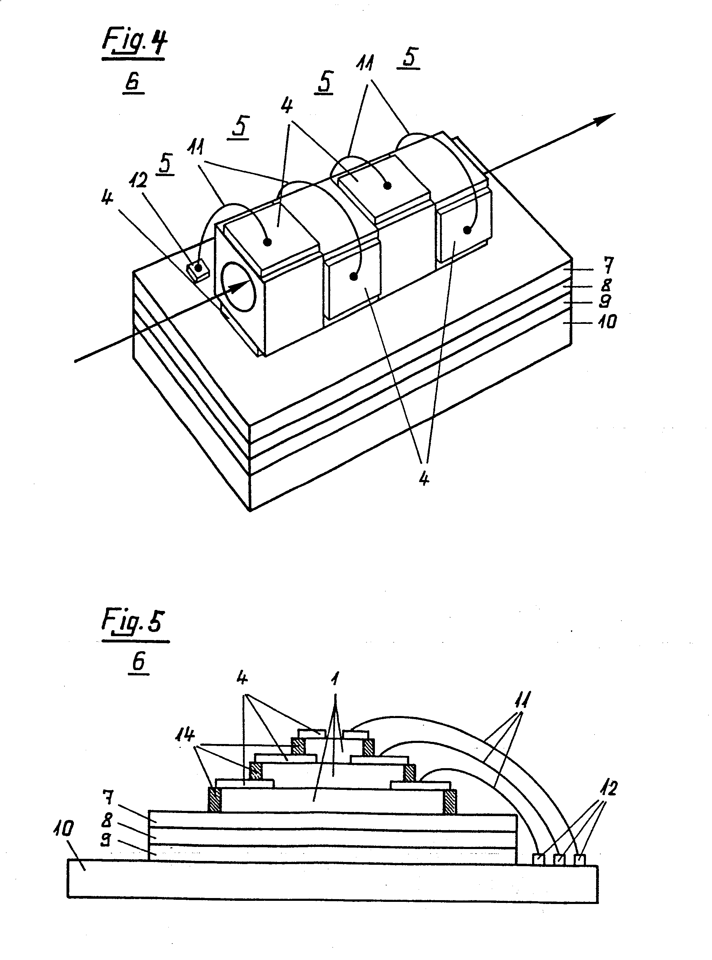

Die Figur 4 zeigt einen anderen Gassensor 6. Mehrere Sensorelemente 5 in Form eines Kubus haben in der Mitte ein Loch,
das durch sämtliche Sensorelemente 5 hindurchgeht und eine
Röhre bildet. Durch diese Röhre wird die zu untersuchende
Luft geführt, die am Röhrenende wieder austritt. Die Strömungsrichtung ist durch Pfeile angedeutet. Die Sensorelemente 5 ändern ihre elektrische Leitfähigkeit, wenn dasjenige Gas an ihrer Oberfläche vorbeizieht, für welches ihr
Ansprechverhalten optimiert wurde. Jedes Sensorelement 5 weist
Elektroden 4 auf, die so angeordnet sind, dass sie sich trotz
der kompakten Anordnung der Sensorelemente 5 nicht gegenseitig
beeinflussen. Die Leitungen 11 verbinden die Elektroden 11
mit den auf der elektrisch isolierenden Schicht 7 angebrachten
Kontaktpunkten 12, die an eine Auswerteschaltung angeschlossen
sind. Eine Heizschicht 8, die zwischen den elektrisch isolierenden Schicht 7, und 9 angeordnet ist, heizt die Sensorelemente 5 auf die gleiche Betriebstemperatur. Die genannte
Anordnung ist auf das hitzebeständige, wärmeisolierende Substrat 10 geklebt. Mit dem Gassensor 6 der Figur 4 können mehr
als nur vier Gase detektiert werden, wenn entsprechend mehr
Sensorelemente 5 angeordnet werden. Die Sensorelemente 5 können auch nach einer optischen Messmethode betrieben werden
und können durch dünne Schichten elektrisch voneinander isoliert sein.

Der gesamte Block aus Sensorelementen ist durch eine in der Figur 4 nicht eingezeichnete gasdichte Umhüllung umschlossen mit Ausnahme von zwei Oeffnungen an den beiden Enden. Dadurch ist es möglich, einen gerichteten Gasstrom durch die Sensoranordnung zu führen. Gasförmige Bestandteile eines Gasgemisches, die spezifisch am ersten Sensorelement verbraucht werden und dort ein elektrisches Signal ergeben, erzeugen keine Signale mehr an den nächsten Sensorelementen. Gasförmige Bestandteile, die erst am zweiten Sensorelement reagieren und dort ein elektrisches Signal erzeugen, ergeben an den folgenden Sensorelementen keine Signale mehr usw.. Durch diese Anordnung ist es möglich, die Bestandteile eines Gasgemischs an separaten Sensorelementen 5, durch eine Widerstandsänderung derselben nachzuweisen. Die Elektroden 4 in der Figur 4 müssen nicht unbedingt aussen angebracht sein, sondern können auch zwischen den einzelnen Sensorelementen 5 vorgesehen sein.

Für eine noch bessere Trennung des zu untersuchenden Gasgemisches, beispielsweise zur getrennten Bestimmung von gasförmigen Verunreinigungen in Luft, kann der in der Figur 4 dargestellte Gassensor 6 zweckmässigerweise an einem Ende, z.B. an der Eintrittsöffnung, eine tiefere Temperatur aufweisen als an der Austrittsöffnung. Gase die schon bei tiefen Temperaturen verbrennen, werden dann bevorzugt am ersten Sensorelement 5 verbrennen und dort eine grosse Aenderung der elektrischen Signale für die Leitfähigkeit ergeben. Gase, die erst bei höheren Temperaturen verbrennen, werden am zweiten Sensorelement, Gase, die bei noch höheren Temperaturen verbrennen, erst am dritten Sensorelement usw. verbrennen. Dadurch können die Gase noch viel selektiver detektiert werden. Wenn die Sensorelemente aus einem hochporösen Metalloxidgel, z.B. aus einem Aerogel, bestehen, so ist das Mittelloch nicht einmal notwendig. Die Ausführung der Figur 4 bezieht sich auch auf solche Fälle.

Die Figur 5 zeigt einen Gassensor 6, der aus mehreren, pyramidenförmig aufgebauten Sensorelementen 5, besteht. Die Elektroden 4 sind über elektrische Leitungen 11 mit den auf dem hitzebeständigen, wärmeisolierenden Substrat 10 befestigten, elektrischen Kontaktpunkten 12 verbunden, die ihrerseits über nicht dargestellte Verbindungsleitungen mit einer Auswerteschaltung verbunden sind. Die Elektroden 4 sind so isoliert, dass sie nur mit dem Material 1 des zugeordneten Sensorelementes und nicht mit dem Material 1 des anderen Sensorelementes Kontakt haben. Die Aussenwände der Sensorelemente sind teilweise mit einer gasdichten Schicht 14 versehen. Daher sind nur die nach oben liegenden Flächen der Sensorelemente gasaktiv. Die Sensorelemente sind auf der elektrisch isolierenden Schicht 7 befestigt, die mit der Heizvorrichtung 8 und der elektrisch isolierenden Schicht 9 verbunden ist. Die gesamte Anordnung ist auf dem hitzebeständigen wärmeisolierenden Substrat 10 befestigt.

Die einzelnen Sensorelemente werden auf die gleiche Betriebstemperatur erhitzt. Durch Verwendung unterschiedlicher Materialien 1 wird jedes Sensorelement auf einen bestimmten Bestandteil des zu untersuchenden Gasgemisches sensibilisiert. Es besteht ausserdem die Möglichkeit, dass z.B. das oberste Sensorelement bei Zimmertemperatur betrieben wird. In diesem Fall wird das oberste Sensorelement thermisch gegen die anderen Sensorelemente isoliert. Ferner können mehr Sensorelemente für den in Figur 5 dargestellten Gassensor verwendet werden, als in der Figur gezeichnet.

Die Kontaktpunkte 12 in den Figuren 2 bis 5 müssen sich nicht unbedingt auf der elektrisch isolierenden Schicht 7 befinden, sie können auch ausserhalb dieser Schicht liegen.

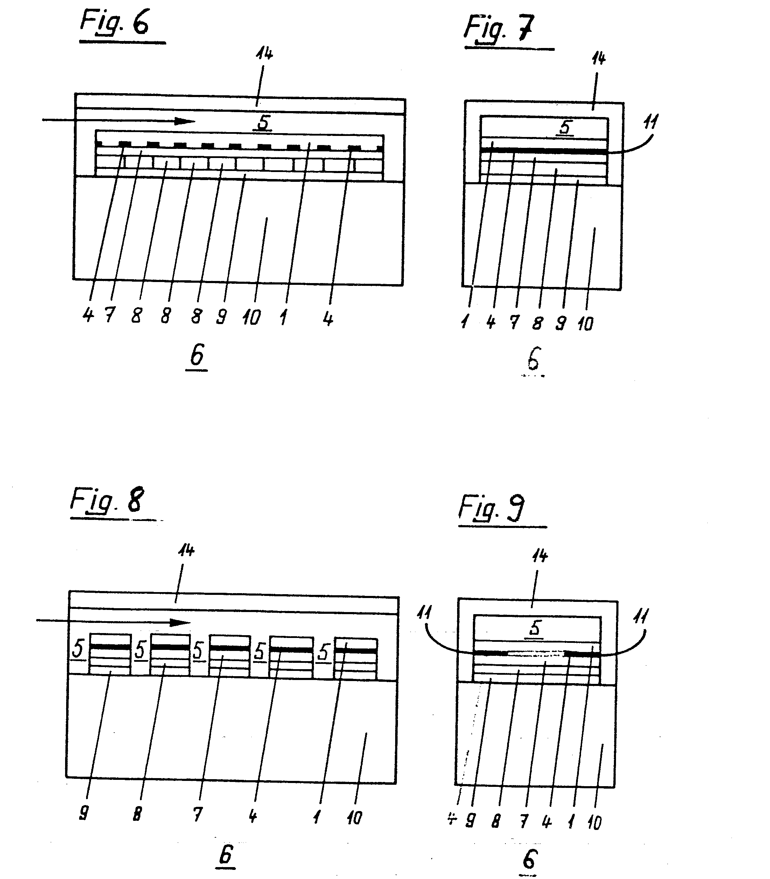

Figur 6 zeigt einen Längsschnitt und Figur 7 einen Querschnitt durch einen Gassensor 6, der aus einer Kombination
mehrerer Sensorelemente besteht, die ein einheitliches Material 1 aufweisen. An der Unterseite dieses Materials 1 befindet sich eine grosse Anzahl von Elektroden 4. Das Sensorelement 5 ist durch eine elektrisch isolierende Schicht 7 auf
der Heizvorrichtung 8 befestigt. Die Heizvorrichtung 8 ganz
links in der Figur 6 heizt die sich darüber befindliche Zone
des Sensorelementes 5 z.B. auf 80°C. Die nächste Heizschicht 8 rechts davon heizt die darüberliegende Zone des
Sensorelementes 5 auf z.B. 90°C usw.. Von links nach rechts
weist das Sensorelement 5 daher eine ansteigende Temperatur
auf. Die einzelnen Heizschichten 8 sind durch eine elektrisch
isolierende Schicht 9 auf einem wärmeisolierenden Substrat
10 befestigt.

Die in den Figuren 6 und 7 dargestellten Ausführungsformen
eines Gassensors 6 zeigen eine gasdichte Schicht 14, die die
Schichten 7, 8, 9 und das Sensorelement 5 so umschliesst,
dass oberhalb des Sensorelementes 5 ein freier Raum bleibt.
Durch diesen freien Raum wird die zu untersuchende Luft von
links nach rechts geleitet, was durch den Pfeil in der Figur 6
angedeutet ist. Dieser Luftstrom kann durch eine nicht eingezeichnete Pumpe erzeugt werden oder auch dadurch, dass der
ganze Gassensor 6 senkrecht angeordnet wird, wobei die Heizvorrichtung 8 mit der tiefsten Temperatur sich unten befindet.
Durch die Kaminwirkung stellt sich dann von selbst ein Luftstrom ein.

In der Figur 7 sind auch noch die elektrischen Leitungen 11
eingezeichnet, die durch die gasdichte Schicht 14 zur Auswerteschaltung führen. Leicht oxidierbare Gase werden nun schon
ganz links, bei tiefer Temperatur, verbrennen und dort grosse
Aenderungen des elektrischen Widerstandes des Sensorelementes 5 ergeben. Schwieriger zu oxidierende Gase werden z.B.
erst in der Mitte des Gassensors 6 bei einer mittleren Temperatur verbrennen und dort eine grosse Aenderung des elektrischen Widerstandes am Sensorelement 5 ergeben. Sehr schwierig oxidierbare Gase hingegen werden erst am rechten, heissen Ende des Gassensors 6 reagieren und dort eine Widerstandsänderung am Sensorelement 5 hervorrufen. Durch diese Anordnung kann also ein Gasgemisch in seine Bestandteile zerlegt und diese Bestandteile separat detektiert werden. Damit wird eine sehr hohe Selektivität des Gassensors erreicht, die durch geeignete mathematische Auswertung der Signale der einzelnen Zonen des Sensorelementes 5 noch beträchtlich gesteigert werden kann. Eine solche Auswertung findet in der nicht gezeichneten Auswerteschaltung statt.

Die Figur 8 zeigt einen Längsschnitt und die Figur 9 einen Querschnitt durch eine weitere Ausgestaltung eines Gassensors 6, der mehrere Sensorelemente 5 enthält, die aus verschiedenem Material 1 bestehen und je zwei Elektroden 4 enthalten. Diese Sensorelemente 5 werden durch separate Heizschichten 8, die sich zwischen elektrisch isolierenden Schichten 7 und 9 befinden und auf einem wärmeisolierenden Substrat 10 befestigt sind, auf individuelle Temperaturen gebracht. So weist das ganz links befindliche Sensorelement 5 z.B. eine Temperatur von nur 65°C auf. Das nächste, rechts davon liegende Sensorelement weist eine Temperatur von 80°C auf. Das nächste eine Temperatur von 100°C usw.. Das ganz rechts liegende Sensorelement hat eine Temperatur von 400°C.

Die in den Figuren 8 und 9 dargestellte Ausführungsform eines Gassensors 6 zeigt auch eine gasdichte Schicht 14, die die Schichten 7, 8, 9 und die Sensorelemente 5 so umschliesst, dass oberhalb der Sensorelemente 5 ein freier Raum offen bleibt. Durch diesen freien Raum wird die zu untersuchende Luft von links nach rechts geleitet, was durch einen Pfeil in der Figur 8 angedeutet ist. Wie schon bei der Figur 6 ausgeführt wurde, kann dieser, vorzugsweise laminare,

**0141089**

Luftstrom entweder durch eine externe, nicht eingezeichnete
Pumpe erzeugt werden oder durch die Kaminwirkung, die entsteht, wenn der Gassensor 6 senkrecht angeordnet wird, wobei
das Sensorelement mit der tiefsten Temperatur sich unten befindet. Die elektrischen Leitungen 11 führen in der Figur 9
durch die gasdichte Schicht 14 zu einer nicht dargestellten
Auswerteschaltung.

Beim Gassensor der Figuren 8 und 9 werden ebenfalls leicht
oxidierbare Gase schon beim ersten sich ganz links befindlichen Sensorelement 5 verbrennen und dort eine grosse Aenderung des elektrischen Widerstandes hervorrufen. Das Material 1
des Sensorelementes 5 ist auch so ausgewählt (es enthält z.B.
einen speziellen Katalysator), dass die leicht oxidierbaren
Substanzen bevorzugt an diesem Sensorelement verbrennen,
wobei die Oxidation durch eine optimale Betriebstemperatur,
die etwas tiefer ist als diejenige des nachfolgenden Sensorelementes, und durch eine geeignete chemische Zusammensetzung
des Materials 1 stark gefördert wird. Da neben den ansteigenden Temperaturen in Richtung des Luftstromes auch die chemische Zusammensetzung der Materialien 1 der Sensorelemente 5
optimal gewählt wird, können die zu detektierenden gasförmigen
Verunreinigungen der Luft äusserst selektiv erfasst werden.
Eine geeignete mathematische Verknüpfung der Signale der einzelnen Sensorelemente 5 kann die Selektivität dieses Gassensors 6 nochmals stark erhöhen.

**0141089**

Patentansprüche

1. Vorrichtung zur selektiven Bestimmung der Bestandteile von Gasgemischen mittels eines Gassensors (6), welcher mehrere Sensorelemente (5) enthält, die bei Einwirkung der zu bestimmenden Mischungsbestandteile ihre elektrische Leitfähigkeit ändern und einer Auswerteschaltung, d a - d u r c h g e k e n n z e i c h n e t, dass die Sensorelemente (5) auf einem hitzebeständigen, wärmeisolierenden Substrat (10) angeordnet sind und die einzelnen Sensorelemente (5) durch Wahl des Materials (1) und/oder der Betriebstemperatur zur Bestimmung unterschiedlicher Bestandteile des Gasgemischs vorgesehen sind.

2. Vorrichtung gemäss Patentanspruch 1, d a d u r c h g e - k e n n z e i c h n e t, dass jedes der Sensorelemente (5) eine separate, zwischen elektrisch isolierenden Schichten (7, 9) angeordnete elektrische Heizvorrichtung (8) aufweist.

3. Vorrichtung gemäss Patentanspruch 1, d a d u r c h g e - k e n n z e i c h n e t, dass die Sensorelemente (5) eine zwischen elektrisch isolierenden Schichten (7, 9) angeordnete, mehreren oder allen Sensorelementen (5) gemeinsame elektrische Heizvorrichtung (8) aufweisen.

4. Vorrichtung gemäss einem der Patentansprüche 1 bis 3, d a d u r c h g e k e n n z e i c h n e t, dass die Heizvorrichtung (8) eine Schicht aus Platin, Iridium oder ihren Legierungen mit einem grossen Temperaturkoeffizienten der elektrischen Leitfähigkeit zur gleichzeitigen Bestimmung der Heiztemperatur ist.

5. Vorrichtung gemäss einem der Patentansprüche 2 bis 4, d a d u r c h   g e k e n n z e i c h n e t, dass die Sensorelemente (5), die Heizvorrichtung (8) und das wärmeisolierende Substrat (10) so angeordnet sind, dass das Gasgemisch in einem gerichteten Strom an den Sensorelementen (5) vorbeigeleitet wird.

6. Vorrichtung gemäss einem der Patentansprüche 1 bis 4, d a d u r c h   g e k e n n z e i c h n e t, dass eine gasdichte Schicht (14) vorgesehen ist, die so angeordnet ist, dass das Gasgemisch in einem gerichteten Strom an den Sensorelementen (5) vorbeigeleitet wird.

7. Vorrichtung gemäss einem der Patentansprüche 5 und 6, d a d u r c h   g e k e n n z e i c h n e t, dass die Sensorelemente (5) in Richtung des Gasstroms gesehen hintereinander angeordnet sind.

8. Vorrichtung gemäss Patentanspruch 7, d a d u r c h   g e - k e n n z e i c h n e t, dass die Sensorelemente (5) einen zusammenhängenden Block mit einer Reihe von Elektroden bilden, wobei in Richtung des Gasstroms ein Temperaturgradient vorhanden ist.

9. Vorrichtung gemäss Patentanspruch 7, d a d u r c h   g e - k e n n z e i c h n e t, dass die Sensorelemente (5) auf eine unterschiedliche Betriebstemperatur aufheizbar sind, wobei vorzugsweise das in Richtung des Gasstroms gesehen vorn liegende Sensorelement (5) eine niedrigere Temperatur aufweist als das dahinter liegende.

0141089

1/3

Fig.1

Fig.2

Fig.3

Fig.4
6

Fig.5
6

_Fig. 6_

14

5

4 7 8 8 8 9 10 1 4

_6_

_Fig. 7_

14

5

11

1 4 7 8 9 10

_6_

_Fig. 8_

14

5 5 5 5 5

9 8 7 4 1 10

_6_

_Fig. 9_

14

11

5

11

4 9 8 7 4 1 10

_6_